# EUROPEAN PATENT SPECIFICATION

(11) **EP 1 136 062 B1**
(45) Date of publication and mention of the grant of the patent: **30.06.2004**
(21) Application number: 01101645.8
(22) Date of filing: 26.01.2001
(51) Int. Cl.: A61K 7/48

(54) **Cosmetic use of compositions comprising sesquiterpene alcohol**
Kosmetische Verwendung von Zusammensetzungen die ein Sesquiterpenalkohol enthalten
Utilisation de compositions cosmétiques comprenant un alcool sesquiterpénique

(30) Priority: 28.01.2000 JP 2000020589
(43) Date of publication of application: 26.09.2001
(73) Proprietor: Kao Corporation, Tokyo 103-8210 (JP)
(72) Inventor: Nojiri, Hiroshi, Kao Corp.Research Laboratories, Haga-gun, Tochigi 321-3497 (JP); Nonomura, Mami, Kao Corp.Research Laboratories, Haga-gun, Tochigi 321-3497 (JP); Hori, Kimihiko, Kao Corp.Research Laboratories, Haga-gun, Tochigi 321-3497 (JP)
(74) Representative: HOFFMANN - EITLE

(56) References cited:
- EP-A- 0 714 655
- WO-A-97/46246
- WO-A-99/33443
- PATENT ABSTRACTS OF JAPAN vol. 1999, no. 01, 29 January 1999 (1999-01-29) & JP 10 279417 A (SHISEIDO), 20 October 1998 (1998-10-20)
- DATABASE WPI Derwent Publications Ltd., London, GB; AN 1999-210725 XP002173447 & JP 11 049646 A (KAO), 23 February 1999 (1999-02-23)
- DATABASE WPI Derwent Publications Ltd., London, GB; AN 1986-085710 XP002173448 & JP 61 033129 A (NITTO ELECTRIC IND), 17 February 1986 (1986-02-17)

## Description

### BACKGROUND OF THE INVENTION

### 1. Field of the Invention

The present invention relates to a cosmetic composition having an effect for alleviating the rough or moisture-lost skin and also an effect for improving the condition of the dry or sensitive skin.

### 2. Description of the Related Art

Natural moisture retaining factors are well known to have a moisture absorbing power as water-soluble components in the stratum corneum, that is, the outermost layer of the skin, and play an important role for the flexibility of the stratum corneum. Particularly those regarded to have the dry skin tend to suffer from various skin troubles because their skin does not have, in the stratum corneum thereof, a sufficient amount of such water soluble components, has lost a moisture absorbing capacity, and inevitably has lowered elasticity or flexibility. Accordingly, it is a common practice to add various moisture retaining components to a flexibility-imparting cosmetic composition in order to impart a large amount of moisture to the stratum corneum and at the same time, to retain the thus-imparted moisture for long hours. Since natural moisture retaining components contain an amino acid, organic acid (carboxylic acid), urea and the like, there has been an attempt to incorporate an amino acid, an organic acid or urea in a cosmetic composition, thereby making up for a lost portion of the water soluble components in the stratum corneum and improving skin functions (Japanese Patent Applications Laid-Open Nos. 19291/1980, 8007/1983, 99315/1987, 178207/1990, and 163129/1993).

In addition, incorporation of a betaine in a cosmetic composition has been proposed, because the betaine has excellent moisture retaining and flexibility imparting effects, increases a turnover rate of the stratum corneum and has an effect for preventing the roughening of the skin (Japanese Patent Applications Laid-Open Nos. 275511/1989 and 92060/1996).

Flexibility imparting effects available from such components are however transient. For those having the dry or sensitive skin, effects for improving their skin roughness or moisture loss of the skin therefore do not last long. Particularly in the case of the sensitive skin, itching or eczema appears even by simple stimulation or skin troubles tend to occur by the repetition of mental stresses. Thus, cosmetics effective for such dry or sensitive skin have not been known yet.

On the other hand, a dermal preparation for external use which contains a sesquiterpene compound such as guaiol or cedrol having a melanogenesis suppressing action is known (Japanese Patent Applications Laid-Open Nos. 36246/1998 and 36247/1998). A dermal composition for external use which contains a sesquiterpene, as a bioactive substance, and a water-soluble polyhydric alcohol is also known (Japanese Patent Application Laid-Open No. 128120/1994). The bioactive substance employed in the above-described composition however is not effective for alleviating the rough or moisture-lost skin, because it exhibits its action while not staying in the epidermis, particularly in the stratum corneum but passing through the skin and being absorbed in the tissue below the skin or moving throughout the body as a blood flow after absorbed in the blood.

EP-0 714 655 A1 describes a procedure to fight bad orders of the body emitted in zones of transpiration by a cosmetic composition, which comprises sodium lactate and sesquiterpene alcohol such as vetiverol.

An object of the present invention is to provide a cosmetic composition highly effective for alleviating the rough or moisture-lost skin even when the skin is dry or sensitive.

### SUMMARY OF THE INVENTION

The present inventors have found a cosmetic composition highly effective for alleviating the skin roughness or moisture loss of the skin of even those having the dry or sensitive skin, thereby regulating their skin condition by the use of a specific moisture retaining agent and a sesquiterpene alcohol component in combination.

In the present invention, there is thus provided the cosmetic use of the combination of the following components (A) and (B):
(A) at least one moisture retaining component selected from the group consisting of amino acids and salts thereof, pyrrolidonecarboxylic acid and salts thereof, lactic acid and salts thereof, urea and betaines; and
(B) at least one sesquiterpene alcohol for the alleviation of rough or moisture-lost skin and for the improvement of the skin condition of dry or sensitive skin.

The cosmetic composition used in the present invention exhibits high effects for improving the skin roughness or moisture loss of the skin and improving the skin condition of the dry or sensitive skin.

### DETAILED DESCRIPTION OF THE PREFERRED EMBODIMENTS

Component (A) to be used in the cosmetic composition used in the present invention is at least one moisture retaining component and is selected from the group consisting of amino acids and salts thereof, pyrrolidonecarboxylic acid and salts thereof, lactic acid and salts thereof, urea and betaines. Examples of the amino acids and salts thereof include amino acids such as lysine, histidine, arginine, ornithine, carnavalin, glutamine, glutamic acid, asparagine, aspartic acid, serine, alanine, glycine, leucine, isoleucine, proline, threonine, valine, methionine, cystine, cysteine, hydroxyproline and phenylalanine, and salts thereof. Among them, basic amino acids such as lysine, histidine and arginine are preferred, with arginine being particularly preferred for flexibility imparting effects. Examples of the salt of the above-exemplified amino acids include sodium salts, potassium salts, calcium salts and magnesium salts.

Examples of the salt of pyrrolidonecarboxylic acid or lactic acid include sodium salts, potassium salts, calcium salts and magnesium salts.

Among these components (A), amino acids and salts thereof, pyrrolidonecarboxylic acid and salts thereof, lactic acid and salts thereof and urea can be used either singly or in combination. Such a moisture retaining component is added to the cosmetic composition used in the present invention in an amount of 0.01 to 60 wt.%, especially 0.05 to 40 wt.% based on the whole composition, with 0.1 to 20 wt.% being more preferred for excellent feeling upon use.

As the betaine serving as Component (A), various N-trialkyl-substituted derivatives of an amino acid can be used. Examples include glycinebetaine, γ-butyrobetaine, homarine, trigonelline, β-alaninebetaine, carnitine, homoserinebetaine, antopreurine, valinebetaine, lysinebetaine, ornithinebetaine, alaninebetaine, taurobetaine, betaine glutamate and phenylalaninebetaine. Among them, glycinebetaine is particularly preferred for its excellent flexibility imparting effects. As such a betaine, commercially available products can be used. For example, "Aminocoat" (trade name; product of Asahi Foods) can be used as glycinebetaine. In the present invention, the above-exemplified betaines may be used either singly or in combination. Although no particular limitation is imposed on the amount of the betaine to be added to the cosmetic composition of the present invention, 0.01 to 10 wt.%, especially 0.1 to 3 wt.% is preferred for attaining sufficient moisture retaining and flexibility imparting effects.

Examples of the sesquiterpene alcohol serving as Component (B) in the cosmetic composition used in the present invention include nerolidol, cedrol, guaiol, vetiverol, santalol and patchouli alcohol. Among them, cedrol is particularly preferred for its effects for improving the rough or moisture-lost skin and regulating the skin condition.

In the present invention, extract, steam distillate or pressed product from a plant containing such a sesquiterpene alcohol can also be employed. Examples of such a plant include cedar, patchouli, sandalwood, vetiver, ginger, cumin, thick-haired codium, pepper, peppermint, rose, jasmine, Japanese valerian, Japanese honeysuckle, thyme, tea and guaiac wood. Among them, cedar, patchouli, sandalwood, vetiver, ginger, pepper, peppermint, rose, Japanese honeysuckle and guaiac wood are particularly preferred. These plants may be extracted, steam distilled or pressed in a manner known *per se* in the art. The extract, distillate or pressed product thus obtained can be used as is or after fractionation or derivation by using a purified oil or by reaction such as acetylation.

Sesquiterpene alcohols may be used either singly or in combination. Use of at least two of them or use of at least two plant extracts containing a terpene component is preferred for further enhancing the skin regulating effects. Component (B) is preferably added in an amount of 1 ppm or greater, especially 0.01 to 20 wt.%, more especially 0.1 to 10 wt.% based on the whole cosmetic composition used in the present invention.

The cosmetic composition of the present invention preferably has a pH of 2 to 6.9, especially 3 to 6.5 for attaining good effects for improving the skin roughness or moisture loss of the skin or for regulating the skin. For the pH adjustment, use of various buffer components is preferred, with formation of a buffer system with the lactate of the above-described component (A) being particularly preferred.

As well as the above-described essential components, components ordinarily employed for cosmetics, quasi-drugs or drugs can be incorporated in the cosmetic composition of the present invention as needed within an extent not impairing the advantages of it. Examples of such a component include sphingosine derivatives as described in Japanese Patent Application Laid-Open No. 85924/1993, guanidine derivatives as described in Japanese Patent Application Laid-Open No. 92054/1996, natural ceramide, substances having an analogous structure to ceramide as described in Japanese Patent Application Laid-Open No. 228048/1987, arginine-containing peptides as described in Japanese Patent Application Laid-Open No. 330572/1995, plant extracts, whitening agents, anti-inflammatory agents, singlet oxygen scavengers, antioxidants, polysaccharides, alcohols, sterols, blood flow promoters, water, ethanol, surfactants, oil components, silicones, fluorine oils, ultraviolet protecting agents, powders, gelling agents, film forming agents, sebum secretion inhibitors, softeners, antiseptics, sequestering agents, colorants and perfumes.

The cosmetic composition used in the present invention can be prepared in a conventional manner and formulated into any form such as solubilized form, emulsified form, powder dispersed and solubilized form, powder dispersed and emulsified form, and powder dispersed oil. It is suited as a skin cosmetic composition, for example, a skin-care cosmetic composition such as lotion, cream, beauty essence or cosmetic oil, or a make-up cosmetic composition such as foundation, powder, lipstick, cheek rouge or eye shadow. Use as an agent or cosmetic composition for improving skin roughness is particularly preferred.

### Examples

### Example 1

Emulsified cosmetic compositions having the components as shown in Tables 1 and 2 were prepared in a conventional manner, respectively and their effects for improving the skin roughness or moisture loss of the skin and also effects for regulating the skin condition were evaluated. The results are collectively shown in Tables 1 and 2.

### (Evaluation method)

### (1) Effects for improving the rough or moisture-lost skin

The roughness or moisture loss of the skin as compared between before and after use of each of the cosmetic compositions twice a day (morning and night) for 1 week was evaluated by a panel of 10 female experts (20 to 30 years old) frequently suffering from the rough or moisture-lost skin. The results are shown in terms of an average value in Tables.

5: A marked improvement compared with the condition before use.

4: A fair improvement compared with the condition before use.

3: A slight improvement compared with the condition before use.

2: Almost no change compared with the condition before use.

1: No change compared with the condition before use

0: Deterioration

(2) The appearing tendency of the roughness or moisture loss of the skin in the daily life as compared between before and after use of each of the cosmetic compositions twice a day (morning and night) for 1 month was evaluated based on the below-described standards by a panel of 10 female experts (20 to 30 years old) conscious of their sensitive skin. The results are shown in terms of an average value in Tables.

5: The appearing tendency of the roughness or moisture loss of the skin shows a drastic decrease, compared with that before use.

4: The appearing tendency of the roughness or moisture loss of the skin decreases largely, compared with that before use.

3: The appearing tendency of the roughness or moisture loss of the skin shows a slight increase, compared with that before use.

2: The roughness or moisture loss of the skin appears at a similar frequency to that before use and it sometimes occurs.

1: The roughness or moisture loss of the skin appears at a similar frequency to that before use and it occurs frequently.

0: The appearing tendency of the roughness or moisture loss of the skin shows an increase, compared with that before use.

**Table 1**

| | invention Compound | | | | Comparative Compound | | |
|---|---|---|---|---|---|---|---|
| | 1 | 2 | 3 | 4 | 1 | 2 | 3 |
| Arginine | 3 | 3 | 3 | 3 | 10 | - | - |
| Patchouli oil *1 | 5 | - | - | 2.5 | - | 5 | - |
| Cedar wood oil*2 | - | 5 | - | 2.5 | - | 5 | - |
| Cedrol | - | - | 5 | - | - | - | - |
| lsostearyl glyceryl ether | 2 | 2 | 2 | 2 | 2 | 2 | 2 |
| Sorbitan monostearate | 2 | 2 | 2 | 2 | 2 | 2 | 2 |
| 2-Octyldodecyl myristate | 10 | 10 | 10 | 10 | 10 | 10 | 10 |
| Squalane | 5 | 5 | 5 | 5 | 5 | 5 | 5 |
| Glycerin | 5 | 5 | 5 | 5 | 5 | 5 | 5 |
| Purified water | Balance | Balance | Balance | Balance | Balance | Balance | Balance |
| Improving degree of rough or moisture-lost skin | 4.5 | 4.6 | 4.8 | 4.9 | 1.8 | 2.5 | 0.8 |
| Skin condition regulator | 4.5 | 4.2 | 4.6 | 5.0 | 2.2 | 1.9 | 1.0 |

| | | | | | | | |
|---|---|---|---|---|---|---|---|
| *1: containing 30 wt.% of patchouli alcohol and 20 wt.% of caryophyllene. | | | | | | | |
| *2: containing 24 wt.% of cedrol. | | | | | | | |

**Table 2**

| | Invention products | | | |
|---|---|---|---|---|
| | 5 | 6 | 7 | 8 |
| Betaine*3 | 0.1 | 0.1 | 0.1 | 0.1 |
| Patchouli oil*1 | 0.5 | - | - | 0.25 |
| Cedar wood oil*2 | - | 0.5 | - | 0.25 |
| Cedrol | - | - | 0.5 | - |
| Isostearyl glyceryl ether | 2 | 2 | 2 | 2 |
| Sorbitan monostearate | 2 | 2 | 2 | 2 |
| 2-Octyldodecyl myristate | 10 | 10 | 10 | 10 |
| Squalane | 5 | 5 | 5 | 5 |
| Glycerin | 5 | 5 | 5 | 5 |
| Purified water | Balance | Balance | Balance | Balance |
| Improving degree of rough or moisture-lost skin | 4.1 | 4.0 | 4.0 | 4.2 |
| Skin condition regulator | 3.7 | 4.1 | 3.6 | 4.4 |

| | | | | |
|---|---|---|---|---|
| *3: glycinebetaine, "Aminocoat" (trade name, product of Asahi Chemical). | | | | |

As is apparent from Tables 1 and 2, it has been found that each of the cosmetic compositions of the present invention comprising Components (A) and (B) exhibits marked effects for alleviating rough or moisture-lost skin and for regulating the condition of the skin, compared with the compositions containing either one of these components.

### Example 2

### Example 2 (Cosmetic lotion)

A cosmetic composition having the below-described components was prepared in a conventional manner.

| (Components) | (wt.%) |
|---|---|
| Vetiver oil (containing 60 wt.% of vetivelol) | 2.0 |
| Sodium pyrrolidonecarboxylate | 1.0 |
| Polyoxyethylene (20) sorbitanmonolaurate ester | 1.5 |
| Glycerin | 2.0 |
| Purified water | Balance |

### Example 3 (O/W type emulsion)

An O/W type emulsion having the below-described composition was prepared in a conventional manner.

| (Components) | (wt.%) |
|---|---|
| Sandalwood oil (containing 73 wt.% of santalol) | 2.0 |
| Cetyl alcohol | 1.0 |
| Vaseline | 2.0 |
| Squalane | 6.0 |
| Dimethylpolysiloxane | 2.0 |
| Glycerin | 2.0 |
| Sodium lactate | 1.0 |
| Polyoxyethylene (10) monoleate ester | 1.0 |
| Glycerol monostearate ester | 1.0 |
| Paraben | 0.2 |
| Purified water | Balance |

### Example 4 (W/O type cream)

A W/O type emulsion having the below-described composition was prepared in a conventional manner.

| (Components) | (wt.%) |
|---|---|
| Guaiac wood oil (containing 64 wt.% of guaiol) | 2.0 |
| Dimethylpolysiloxane | 10.0 |
| Methylphenyl polysiloxane | 3.0 |
| Octamethylcyclotetrasiloxane | 12.0 |
| Polyoxyalkylene-modified silicone | 5.0 |
| 1,3-Butylene glycol | 6.0 |
| Urea | 1.2 |
| Paraben | 0.2 |
| Perfume | Trace |
| Purified water | Balance |

It has been found that cosmetic composition available in any one of Examples 2 to 4 was highly effective for alleviating the rough or moisture-lost skin and had effects for regulating the skin condition such as dry or sensitive skin.

## Claims

1. Cosmetic use of the combination of the following components (A) and (B):
(A) at least one moisture retaining component selected form the group consisting of amino acids and salts thereof, pyrrolidonecarboxylic acid and salts thereof, lactic acid and salts thereof, urea and betaines; and
(B) at least one sesquiterpene alcohol,
for the alleviation of rough or moisture-lost skin and for the improvement of the skin condition of dry or sensitive skin.

2. The use according to claim 1, wherein the sesquiterpene alcohol as component (B) is selected from the group consisting of nerolidol, cedrol, guiaol, vetiverol and patchouli alcohol.

## Patentansprüche

1. Kosmetische Verwendung der Kombination der folgenden Komponenten (A) und (B):
(A) zumindest einer feuchtigkeitsbeibehaltenden Komponente, ausgewählt aus der Gruppe bestehend aus Aminosäuren und Salzen davon, Pyrrolidoncarbonsäure und Salzen davon, Milchsäure und Salzen davon, Harnstoff und Betainen; und
(B) zumindest einen Sesquiterpenalkohol,
zur Linderung von rauher Haut oder Haut, die Feuchtigkeit verloren hat, und zur Verbesserung des Hautzustands von trockener oder empfindlicher Haut.

2. Verwendung nach Anspruch 1, worin der Sesquiterpenalkohol als Komponente (B) ausgewählt ist aus der Gruppe bestehend aus Nerolidol, Cedrol, Guiaol, Vetiverol und Patchoulialkohol.

## Revendications

1. Utilisation cosmétique de la combinaison des composants (A) et (B) suivants :
(A) au moins un composant retenant l'humidité choisi dans le groupe constitué par les acides aminés et leurs sels, l'acide pyrrolidonecarboxylique et ses sels, l'acide lactique et ses sels, l'urée et les bétaïnes ; et
(B) au moins un alcool sesquiterpénique,
pour l'apaisement d'une peau rugueuse ou qui n'est plus hydratée et pour l'amélioration de l'état de peau d'une peau sèche ou sensible.

2. Utilisation selon la revendication 1, dans laquelle l'alcool sesquiterpénique comme composant (B) est choisi dans le groupe constitué par le nérolidol, le cédrol, le guiaol, le vétivérol et l'alcool de patchouli.
